# EUROPEAN PATENT APPLICATION

(11) **EP 3 756 648 A1**
(43) Date of publication of application: **30.12.2020**
(21) Application number: 19182807.8
(22) Date of filing: 27.06.2019
(51) Int. Cl.: A61K 9/00, A61K 31/00, A61K 38/06, A61K 39/00

(54) **IMPROVED VACCINE FORMULATIONS**

(71) Applicant: IMNATE SARL, 8009 Strassen (LU)
(72) Inventor: SAINT-REMY, Jean-Marie, 1390 Grez-Doiceau (BE)
(74) Representative: Office Kirkpatrick

(57) **Abstract**

A pharmaceutically compatible antioxidant for use in the treatment or the prevention of an unwanted immune response, the corresponding pharmaceutical and vaccine compositions, and the corresponding clinical and *ex-vivo* applications.

SEQUENCE LISTING

<110>  Imnate

<120>  Improved vaccine formulations

<130>  pbf1761EP00

<160>  8

<170>  PatentIn version 3.5

<210>  1
<211>  20
<212>  PRT
<213>  Artificial sequence

<220>
<223>  myelin O epitope

<400>  1

Ser Ser Lys Arg Gly Arg Gln Thr Pro Val Leu Tyr Ala Met Leu Asp
1               5                   10                  15

His Ser Arg Ser
            20

<210>  2
<211>  8
<212>  PRT
<213>  Artificial Sequence

<220>
<223>  Ovalbumin epitope

<400>  2

Ser Ile Ile Asn Phe Glu Lys Leu
1               5

<210>  3
<211>  11
<212>  PRT
<213>  Artificial sequence

<220>
<223>  nAchR CD1d epitope

<400>  3

Phe Ala Ile Val Lys Phe Thr Lys Val Leu Leu
1               5                   10

<210>  4
<211>  17
<212>  PRT
<213>  Artificial sequence

<220>
<223>  alpha gliadine epitope

<220>
<221>  MISC_FEATURE
<222>  (4)..(4)
<223>  Deamidation

<400>  4

Gln Leu Gln Pro Phe Pro Gln Pro Glu Leu Pro Tyr Pro Gln Pro Gln
1               5                   10                  15

Ser

<210>  5
<211>  17
<212>  PRT
<213>  Artificial sequence

<220>
<223>  Daeminated gliadine pos Q7E

<220>
<221>  MISC_FEATURE
<222>  (7)..(7)
<223>  Q7E deamination

<400>  5

Pro Phe Pro Glu Pro Glu Glu Pro Glu Leu Pro Tyr Pro Gln Pro Gln
1               5                   10                  15

Ser

<210>  6
<211>  2351
<212>  PRT
<213>  Homo sapiens

<400>  6

<210>  7
<211>  228
<212>  PRT
<213>  artificial sequence

<220>
<223>  Heavy chain Fab fragment anti TNF alpha

<400>  7

Gln Val Gln Leu Val Gln Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Val Met Asn Trp Val Arg Gln Ala Pro Gly Lys Gly Leu Glu Trp Val
        35                  40                  45

Ser Gly Ile Ser Gly Gly Gly Gly Ser Thr Tyr Tyr Ala Asp Ser Val
    50                  55                  60

Lys Gly Arg Phe Thr Ile Ser Arg Asp Asn Ser Met Asn Thr Leu Tyr
65                  70                  75                  80

Leu Gln Met Asn Ser Leu Arg Ala Glu Asp Thr Ala Val Tyr Tyr Cys

85                  90                  95

Ala Lys Asp Leu Ser Asn Arg Leu Ser Gly Gly Gly Thr Phe Asp Ile
            100                 105                 110

Trp Gly Gln Gly Thr Met Val Thr Val Ser Ser Gly Ser Ala Ser Ala
        115                 120                 125

Pro Thr Leu Phe Pro Leu Val Ser Cys Glu Asn Ser Pro Ser Asp Thr
    130                 135                 140

Ser Ser Val Ala Val Gly Cys Leu Ala Gln Asp Phe Leu Pro Asp Ser
145                 150                 155                 160

Ile Thr Phe Ser Trp Lys Tyr Lys Asn Asn Ser Asp Ile Ser Ser Thr
                165                 170                 175

Arg Gly Phe Pro Ser Val Leu Arg Gly Gly Lys Tyr Ala Ala Thr Ser
            180                 185                 190

Gln Val Leu Leu Pro Ser Lys Asp Val Met Gln Gly Thr Asp Glu His
        195                 200                 205

Val Val Cys Lys Val Gln His Pro Asn Gly Asn Lys Glu Lys Asn Val
    210                 215                 220

Pro Leu Pro Val
225

<210>  8
<211>  213
<212>  PRT
<213>  Artificial sequence

<220>
<223>  Light chain Fab fragment anti TNF alpha

<400>  8

Asp Ile Glu Leu Thr Gln Ser Pro Ser Thr Leu Ser Ala Ser Val Gly
1               5                   10                  15

Asp Arg Val Thr Ile Thr Cys Arg Ala Ser Gln Ser Leu Asn Asn Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Ala Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asn Ser Pro Trp Thr
                85                  90                  95

Phe Gly Gln Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Leu Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

## Description

### Field of the invention

The present invention relates to new vaccination compositions, enriched into reducing compounds, useful for the treatment of autoimmune diseases and diseases associated with chronic tissue inflammation, or to be administered together with biological peptides used in replacement therapies.

### BACKGROUD OF THE INVENTION

Autoimmune diseases are characterized by the production of antibodies and activation of lymphocytes directed towards self-antigens, leading to the progressive loss of function of the target organ.
Although there is clear evidence for the pathogenic role of autoantibodies and autoreactive immune cells in the triggering and maintenance of autoimmune diseases, supported by the relative efficacy of therapies based on non-specific immunosuppression or administration of antibodies targeting cytokines, there is no cure for such diseases. This, combined to the steadily raising incidence of autoimmunity, constitutes a highly significant unmet medical need. Indeed, strategies by which it would become possible to suppress the autoimmune response without affecting the overall immune system are much desired.

Currently, a limited number of strategies have been defined in an attempt to selectively suppress the autoimmune response.
However, these approaches are very complex in practice, sometimes associated only to a transient effect and the demonstration of their significant usefulness is sometimes lacking.

The patent application WO2008/017517 A1 (Immunogenic peptides and their use in immune disorders) describes peptides and methods wherein class II MHC epitopes containing a redox (thioreductase) motif C-X-X-C (wherein C stands for cysteine and X for any aminoacid) are used for eliciting epitope-specific CD4+ T cells with cytolytic properties. Elimination by cytolysis of the activating APC and of bystander T cells is said to be efficient for the treatment of immune disorders, and in particular autoimmune and allergic diseases. These peptides contain a thioreductase motif which is attached by a covalent amide linkage (peptide bond), on either side of the epitope sequence, with or without an aminoacid linker. Due to the open end structure of MHC class II molecules, it is indeed possible to use peptides much longer than what would be allowed if length would be limited by the sequence inserted into the cleft of the class II element.

Extending from the field of overt autoimmune diseases, there is a number of pathological conditions characterized by chronic inflammation, but wherein a specific autoantigen has not been convincingly demonstrated. One example is obesity. Adipose tissue chronic inflammation is prominent in such condition and recent evidence strongly suggests that such inflammation is inversely related to the presence of T cells with suppressive properties.
Beyond auto-immune diseases, immune reaction to (injected) biological molecules represents also a major problem, not totally solved so far.

T lymphocytes remain the key cells at the start of an autoimmune response or of tissue specific inflammation. Antigen-specific T cells are divided in three separate lineages, defined by the restriction element by which they are activated. CD4+ T cells are elicited in the context of presentation by MHC class II complexes, CD8+ T cells are activated through MHC class I presentation and natural killer T (NKT) cells are activated by presentation by the MHC-like CD1 molecule.
Antigen-presenting cells (APCs) when exposed to an antigen, or an epitope of it, process the antigen and expose it at their surface for specific T cell activation in a scenario which is classically described in 3 steps: (1) contact between a T cell via its antigen-specific receptor (CD3) with the antigen epitope as processed by the APC and presented in the context of an MHC molecule (signal 1); (2) interaction between the costimulatory signals expressed at the APC surface and their respective ligand or receptor at the T cell surface (signal 2); and, (3) production of soluble factors including cytokines and chemokines by the APC (signal 3).
In the setting of autoimmune diseases, or tissue-associated chronic inflammation, a vaccination strategy aiming at suppressing the unwanted response takes these signals into account. In short, intrinsic tolerance is obtained primarily in the absence of an adjuvant, whilst extrinsic tolerance is obtained by manipulating the cytokine milieu under which activation occurs.
However, these methods are not versatile or potent enough to treat complex diseases.
The efficacy of biologicals administered as therapy of an increasing number of diseases is often limited by emergence of an immune response resulting in either the neutralization of the therapeutic effect, an increase in clearance rate and/or diverse modes of hypersensitivity reactions, including serum sickness, anaphylactic reactions and cutaneous eruptions. Preventing such responses would reduce side effects, decrease doses and therefore cost of biologicals, and allow a higher number of patients to benefit from such biologicals.

### Brief description of the invention

The present patent application relates to a pharmaceutically compatible antioxidant for use in the treatment or the prevention of an unwanted immune response.

Preferably, this pharmaceutically compatible antioxidant is present (incorporated) in a pharmaceutical composition (or in a pharmaceutical kit of parts) further comprising a pharmaceutical (injectable) peptide molecule, wherein this pharmaceutical (injectable) peptide molecule is preferably selected from the group of antigens associated to autoimmune and/or chronic inflammatory diseases epitopes, antibodies, biologicals for replacement therapies (lysosomial enzymes, cytokines, hormones, coagulation factors) and epitopes being part of the said biologicals for replacement therapies.
When the pharmaceutical peptide risks to be affected by the antioxidant, for instance if the pharmaceutical peptides comprises important disulphide bridges, the pharmaceutical antioxidant is incorporated in mild conditions, to not irreversibly affect the pharmaceutical peptide. One way to achieve this is to incorporate the antioxidant in a pharmaceutical kit of parts; the pharmaceutical peptide and the pharmaceutical antioxidant being mixed just before the administration to a patient.
Preferably, this pharmaceutically compatible antioxidant (possibly with the pharmaceutical peptide molecule) is for use in the treatment of autoimmune diseases or in inducing tolerance to peptide-based biologicals used in replacement therapies.
Advantageously, this pharmaceutically compatible antioxidant, or this pharmaceutical composition is for administration by the subcutaneous route.
A related aspect of the present invention is a vaccine composition comprising a peptide-based antigen and a pharmaceutically compatible antioxidant.
Preferably, this vaccine composition further comprises a vaccine adjuvant, more preferably selected from the group consisting of bacterial lipopolysaccharides, CpG oligonucleotides, and aluminium hydroxide.
Advantageously, this vaccine composition (comprising a peptide-based antigen and a pharmaceutically compatible antioxidant) is for use in the treatment of autoimmune diseases, preferably selected from the group consisting of type 1 diabetes, chronic inflammatory demyelinating neuropathies (such as multiple sclerosis), diseases of the neuro-muscular junction (such as myasthenia gravis), diseases of the thyroid (such as Hashimoto's and Grave's diseases), inflammatory diseases of the bowel including Crohn's disease, ulcerative rectocolitis and celiac disease.
Preferably, this vaccine composition is for local injection.
Preferably, the pharmaceutically compatible antioxidant (present in this vaccine composition) is in an amount sufficient for imparting reducing conditions in the extracellular medium of the (local) injection site.
A related aspect of the present invention is an *ex vivo* method for eliciting suppressive antigen-specific T lymphocytes being CD4, CD8 and/or NKT comprising the step of putting *ex vivo* T lymphocyte in contact to a specific antigen, under reducing conditions and preferably selecting the treated lymphocytes with higher surface expression of a molecule selected from the group consisting of TIGIT, DLL4 and CTLA2, and/or with higher secretion of a molecule selected from the group consisting of IL-13, IL-10, prostaglandin E2, TGF-beta, amphiregulin, MMP9 and ADAM33.
Conversely, the present invention also covers T lymphocytes obtainable by such *ex vivo* method, preferably in the form of a pharmaceutical composition.
Another related aspect of the present invention is a method for the treatment of an autoimmune disease or a chronic inflammatory disease (of a specific tissue) affecting a mammalian patient, preferably selected from the group consisting of type 1 diabetes, chronic inflammatory demyelinating (poly)neuropathies (such as multiple sclerosis), diseases of the neuro-muscular junction (such as myasthenia gravis), diseases of the thyroid (such as Hashimoto's and Grave's diseases), inflammatory diseases of the bowel including Crohn's disease, ulcerative rectocolitis and celiac disease, obesity or of an unwanted immune reaction to a peptidic biological molecule administered to a patient, comprising the step of locally administering to this mammalian patient an antioxidant compound (a pharmaceutically acceptable antioxidant compound) together with an epitope designed from this autoimmune disease or with this peptidic biological molecule.
Another related aspect of the present invention is a method to prevent and/or to treat an adverse immune response in a patient towards an administered biological agent, and needing this biological agent comprising the steps of:
- identifying patients developing an adverse immune response towards a biological agent or at risk of developing an adverse immune response towards a biological agent,
- identify an epitope from this biological agent causing, or risking to cause, this adverse immune response,
- incorporating this epitope in a pharmaceutical composition comprising an antioxidant compound,
- administering this pharmaceutical composition comprising an antioxidant compound to this patient, possibly of repeating the step of administering this pharmaceutical composition comprising an antioxidant compound to this patient,
- administering this biological agent to this patient.

Preferably, a vaccine adjuvant is added to the pharmaceutical composition.
Preferably, this (pharmaceutically-compatible) antioxidant compound is present at a concentration comprised between 0.1 µM and 5 mM, preferably between 0.3 µm and 1 mM, more preferably between 1 µM and 0.3 mM, still more preferably between 3 µM and 100 µM, or between 5 µM and 50 µM.
Preferably, this (pharmaceutically-compatible) antioxidant compound is selected from the group consisting of N-acetyl cysteine (including its salts), glutathione, thioredoxin, thioredoxin derivatives, glutaredoxin, peroxiredoxin and gamma interferon-inducible lysosomal thiol reductase (GILT), and mixtures thereof and, preferably, this antioxidant further comprises NADH and/or NADPH, advantageously at a concentration comprised between 0.1 µM and 5 mM, preferably between 0.3 µm and 1 mM, more preferably between 1 µM and 0.3 mM, still more preferably between 3 µM and 100 µM, or between 5 µM and 50 µM, and advantageously further comprises thioreductase.

### DETAILED DESCRIPTION OF THE INVENTION

The inventor has pioneered several methods to fine-tune the immune response, particularly by the specific targeting of T cell lineages CD4, CD8 and or NKT.
The inventor has made the unexpected discovery that the addition of a reducing compound (at least strong enough to reduce disulphide bridges) to a composition for local injection comprising a peptide, and even a vaccine adjuvant, which has been shown by the inventor to boost the efficacity of the present invention, has allowed to elicit a specific immune protection, which is particularly advantageous to treat autoimmune and/or inflammatory diseases, or to use together with peptide biologicals for replacement therapies. The local injection of the biological peptide (here, without an adjuvant) for replacement therapies (and thus of the antioxidant) can be subcutaneous, which is surprising: even in the absence of an adjuvant the risk of eliciting an immune response there is real, due to the high density of Antigen-Presenting Cells (APCs) in the subcutaneous space. The present invention thus allows for a more convenient administration of biologicals (in the form of peptides) to a patient and/or of an improved efficacity over the time, further to specifically treat autoimmune or inflammatory diseases with almost no side-effects.

A first aspect of the present invention is therefore a pharmaceutically compatible antioxidant (at least able to reduce disulphide bridges of peptides) for use in the treatment or the prevention of an unwanted immune response.
Preferably the pharmaceutically compatible antioxidant is present (administered to a patient) at a concentration comprised between 0.1 µM and 5 mM, preferably between 0.3 µm and 1 mM, more preferably between 1 µM and 0.3 mM, still more preferably between 3 µM and 100 µM, or between 5 µM and 50 µM.
The antioxidant is preferably selected from the group consisting of N-acetyl cysteine, glutathione, thioredoxin, thioredoxin derivatives, glutaredoxin, peroxiredoxin and gamma interferon-inducible lysosomal thiol reductase (GILT), and mixtures thereof.
Preferably, NADH and/or NADPH, advantageously at a concentration comprised between 0.1 µM and 5 mM, preferably between 0.3 µm and 1 mM, more preferably between 1 µM and 0.3 mM, still more preferably between 3 µM and 100 µM, or between 5 µM and 50 µM is further present to boost the effect of the antioxidant compound. Possibly, the thioreductase enzyme is further added, especially when NAD(P)H is present.
Preferably, this pharmaceutically compatible antioxidant is present in a composition or in a pharmaceutical kit of parts further comprising a pharmaceutical peptide molecule, or administered together with such composition comprising a pharmaceutical peptide molecule.
This pharmaceutical peptide molecule is preferably selected from the group of epitopes (for use in vaccination, such as in vaccination to inhibit unwanted immune response, for instance an autoantigen, or antigens associated to chronic inflammatory diseases (for instance associated to a specific tissue), including obesity), antibodies, biologicals for replacement therapies (lysosomial enzymes, cytokines, hormones, coagulation factors, etc).
The size of the peptide can range from a few amino acids to much more than 1000 amino acids. Indeed, some of the peptides of the present invention are epitopes, meaning a size usually ranging between 7 and 50 amino acids, or biological molecules, such as coagulation Factor VIII, with a size of 2300 amino acids or antibodies.
This pharmaceutically compatible antioxidant and/or this pharmaceutical composition is advantageously suitable and/or adapted for administration by the subcutaneous route (subcutaneously).
This pharmaceutically compatible antioxidant is advantageous for use in the treatment of autoimmune diseases or in inducing tolerance to peptide-based biologicals used in replacement therapies.
For this approach, advantageously, a vaccine adjuvant can be added to synergize with the induction of the tolerance, which, without the present invention, is paradoxical, since vaccine adjuvants are used to boost an immune response.
Among the autoimmune diseases treated by the antioxidant ((injectable) pharmaceutical composition) of the present invention, are type 1 diabetes, chronic inflammatory demyelinating polyneuropathies and multiple sclerosis, diseases of the neuro-muscular junction (such as myasthenia gravis), diseases of the thyroid (such as Hashimoto's and Grave's diseases), inflammatory diseases of the bowel including Crohn's disease, ulcerative rectocolitis and celiac disease, the invention also encompasses the treatment of unwanted inflammatory status following trauma or ischemic event, as well as chronic inflammation (of a specific tissue) associated to an unwanted response to an antigen, including obesity.
More generally, the autoimmune diseases treated by the antioxidant ((injectable) pharmaceutical composition) of the present invention are :
- multisystem diseases: rheumatoid arthritis, polymyositis and dermatomyositis;
- endocrine diseases: thyroiditis, type 1 diabetes, adrenalitis, polyendocrine syndromes, hypophysitis
- blood diseases: hemolytic anemia, thrombocytopenic purpura, neutropenia, aplastic anaemia, anti-phospholipid syndrome, coagulation disorders;
- neurologic diseases: multiple sclerosis, peripheral neuropathies, ocular diseases, inner ear disease, myasthenia gravis;
- intestinal diseases: Crohn's disease, ulcerative colitis, celiac disease, primary biliary cirrhosis, primary sclerosing cholangitis, gastritis and pernicious anaemia;
- cutaneous diseases: pemphigus, pemphigoid, alopecia, vitiligo, psoriasis, urticaria
- kidney diseases: Goodpasture's disease, ANCA-associated glomerulonephritis;
- cardiac and pulmonary diseases: myocarditis, necrotizing arteritis, vasculitides;
- paraneoplastic diseases.

Conversely, an increasing number of biologicals are used in a large number of diseases; however, a sizable cohort of patients builds, soon or late, an immune response towards these peptidic biologicals, which reduces the efficacity of the treatment, or forces to stop it.
As a consequence of the findings of the inventor, the antioxidant of the present invention thus synergizes with such biologicals in blocking such adverse immune response of the patient towards this biological peptidic molecule.
In this associated aspect, the present invention is even applicable to biologicals which are administered by the subcutaneous or intramuscular route.
The combination of biologicals with antioxidant can be achieved in two ways: either both are administered together, or there is firstly the vaccination step, where an epitope from the biological (e.g. as short as 7 amino acids, or a much bigger molecule, bugger than 20, 50, 100, 200, 500 or even 1000 amino acids) is administered together with the antioxidant, so as to turn down an (established) adverse immune response towards this biological, which is followed by the administration of the biological (possibly together with the antioxidant, to ensure the lowest possible adverse immune response). Caution is taken for biologicals carrying disulphide bridges, so that the pharmaceutical antioxidant does not irreversibly break them down. One convenient way to avoid this is to keep the two compounds separated (e.g. in two different vials of a kit of part), until the administration to a patient.

Among the biologicals, i.e. the injectable biologicals, to be used together with the antioxidant compound of the present invention are:
- replacement therapy for coagulation or fibrinolytic defects, including factor VIII, factor IX and staphylokinase;
- hormones such as growth hormone or insulin;
- cytokines and growth factors such as interferons, GM-CSF and G-CSF;
- antibodies for the modulation of immune responses, including anti-CD3, anti-CD4 and anti-CD20 antibodies, anti-cytokine or cytokine receptor antibodies, and anti-checkpoint inhibitors;
- erythropoietin in renal insufficiency;
- lysosomal enzymes used for lysosomal storage diseases;
- viral vectors used for gene therapy ;
- nucleases used for gene editing.

Another related aspect of the present invention is a vaccine composition comprising a peptide-based antigen and a pharmaceutically compatible antioxidant (at least able to reduce disulphide bridges).
This vaccine composition is advantageously for use in the treatment of autoimmune diseases (as mentioned here above) and/or for the treatment of chronic inflammatory diseases, including chronic inflammatory diseases of a specific tissue, preferably selected from the group consisting of type 1 diabetes, chronic inflammatory demyelinating (poly)neuropathies (such as multiple sclerosis), diseases of the neuro-muscular junction (such as myasthenia gravis), diseases of the thyroid (such as Hashimoto's and Grave's diseases), inflammatory diseases of the bowel including Crohn's disease, ulcerative rectocolitis and celiac disease.
The vaccine composition is preferably for a local (subcutaneous) injection; hence the pharmaceutically compatible antioxidant is in an amount sufficient for imparting reducing conditions in the extracellular medium of the injection site and/or to keep free thiol residues (i.e. reduce disulphide bridges) in the immune synapse.
Preferably the pharmaceutically compatible antioxidant is present in this vaccine composition at a concentration comprised between 0.1 µM and 5 mM, preferably between 0.3 µm and 1 mM, more preferably between 1 µM and 0.3 mM, still more preferably between 3 µM and 100 µM, or between 5 µM and 50 µM.
The antioxidant are preferably selected from the group consisting of glutathione, thioredoxin, thioredoxin derivatives, glutaredoxin, peroxiredoxin and gamma interferon-inducible lysosomal thiol reductase (GILT), and mixtures thereof.
Preferably, NADH and/or NADPH, advantageously at a concentration comprised between 0.1 µM and 5 mM, preferably between 0.3 µm and 1 mM, more preferably between 1 µM and 0.3 mM, still more preferably between 3 µM and 100 µM, or between 5 µM and 50 µM is added in this composition to boost the effect of the antioxidant described here above.
Possibly, thioreductase enzyme is added to this composition, especially when NAD(P)H is present.
Preferably, this vaccine composition further comprises a vaccine adjuvant.

In the context of the present invention, the term "vaccine adjuvant" preferably refers to molecules acting on receptors of immune cells, for instance the pattern recognition receptors. Among preferred vaccine adjuvants are cristals, such aluminium hydroxide and urea, and Toll-like receptors activators, such as lipopolysaccharides (LPS), CpG oligonucleotides, RNA, including dsRNA, or even DNA. Preferably, oils and emulsifying agents, which are sometimes used in vaccination, are not considered as vaccine adjuvants in the context of the present invention. More preferably, the compositions of the present invention are not in the form of water-in-oil or oil-in-water emulsion.

Still another aspect of the present invention is an *ex vivo* method for eliciting suppressive antigen-specific T lymphocytes being CD4, CD8 and/or NKT comprising the step of putting *ex vivo* T lymphocytes in contact to a specific antigen, under reducing conditions, as well as the T lymphocytes obtainable by such method or the pharmaceutical composition comprising such T lymphocytes.

An example of the properties of cells obtained (either *in vivo* through vaccination, or *ex vivo*) by the present invention is provided:
CD4+ T cells obtained by vaccination according to the present invention, with addition of a reducing agent or combination of such agents, do not express the FoxP3 transcription factor and present a number of characteristics endowing them with one or several (preferably at least two) of the following properties:
- reduction of inflammation by production of IL-13 acting on monocytes to reduce the production of IL-6, IL-1a and LIF;
- attracting and conditioning myeloid cells with regulatory properties, by production of Arginine 1;
- generating regulatory T cells by production of IL-10, prostaglandin E2 and TGF-beta;
- providing prostaglandin E2 as a substrate for the function of regulatory T cells in suppressing conventional T cells activation;
- participating in tissue repair by production of amphiregulin;
- production of metalloproteinase such as MMP9 and ADAMs such as ADAM33 with anti-inflammatory, pro-angiogenesis and tissue repair properties;
- production of chitinase like proteins, such as chitinase 3-like-3, or products of equivalent genes in humans, with anti-inflammatory properties exerted on macrophages (M2 conversion) and activation of repair mechanisms and tissue regeneration.

In addition, such cells express a variable number of surface molecules involved in suppressive functions, including TIGIT, DLL4 and CTLA2.

### Examples

### Example 1.

### Induction of suppressive CD4+ T cells for the treatment of chronic inflammatory demyelinating polyneuropathies (CIDP)

Myelin protein zero (0) (myelin 0) is expressed in the peripheral nervous system. Autoimmune reactivity towards myelin 0 is responsible for the development of chronic inflammatory demyelinating polyneuropathies (CIDP) and involves autoreactive CD4+ T lymphocytes.

Mice of the NOD strain (females mainly) are susceptible to spontaneous CIPD mimicking human pathology in the B7.2 KO substrain, but the disease develops within a few weeks when active immunization is carried out. One of the main myelin 0 epitopes associated with CD4+ T cell activation is located in the 180-199 carboxyterminal end of the protein.

A peptide of sequence SSKRGRQTPVLYAMLDHSRS (SEQ ID NO:1) is produced by chemical synthesis.

A vaccination formula is prepared using 100 µg of peptide of SEQ ID NO:1 mixed with aluminium hydroxide and addition of 50 µM of glutathione.

This formula is injected subcutaneously on 4 occasions at a week interval in a group of B7.2 KO female NOD mice. A control group of B7.2 KO female NOD mice is injected by 100 µg of peptide mixed with aluminium hydroxide.

Mice are followed regularly for signs of neuropathy including the development of flaccid tail and extent and intensity of paresis. Six weeks after the last injection a final evaluation for signs of neuropathy is carried out and the mice are sacrificed for evaluation of histological signs of neuropathy and characterization of T lymphocytes.

It is shown that mice vaccinated with the peptide formulation containing glutathione do not show any sign of neuropathy, while 100 % of the control mice vaccinated without addition of glutathione present such signs.

Sections of the sciatic nerve are prepared for histological examination after fixation in formaldehyde. A cellular infiltrate is seen after staining with hematoxilin and eosin concentrating around nerve terminal ends. Scores from 0 to 3 are established corresponding to the intensity of the infiltrates. Staining with an anti-CD3 antibody identifies T lymphocytes. Strikingly, an averaged score of 1 for cell infiltration was calculated for mice treated with the glutahione-containing formulation, while a score of 3 was established in all mice treated with the vaccination formulation without glutathione. Demyelination was evaluated on sections stained with Luxol fast blue. Virtually no myelin segmentation was observed in the glutathione group, while such segmentation was observed in all nerve sections obtained from control mice.

CD4+ splenocyte T cells were prepared from each group and tested in culture for activation with the peptide of SEQ ID N0:1. In the control group, Th1 cells specific for the peptide are obtained, as characterized by production of IFN-γ and expression of the Tbet (Tbx21) transcription factor. By contrast, in the group of mice treated with glutathione, the obtained T cells are characterized by expression of effector memory cells (CD62L(-)) and surface markers including AREG (amphoregulin), TIGIT and DLL4. At transcription level, cells are Foxp3(-), IL-10+, IL-13+ and PGE2+.

It is therefore concluded that addition of glutathione into the vaccination formulation is sufficient as the elicit a population of T cells endowed with suppressive and anti-inflammatory properties, able to accumulate in tissues where they exert anti-inflammatory and healing properties.

### Example 2

### Induction of suppressive CD8+ T cells in a model of type 1 diabetes

Type 1 diabetes in humans is characterized by the presence of class I-restricted CD8+ T cells activated by presentation of insulin epitopes, and exerting a cytotoxic activity destroying islet beta cells. As the spontaneous model of type 1 diabetes in the mouse (NOD strain) is essentially driven by class II-restricted CD4+ T cells, an animal model was used in which ovalbumin is expressed in islets under the promotor of rat proinsulin (RIP). OT-I cells carrying a transgenic receptor for a class I-restricted ovalbumin epitope (thereby classifying as CD8+ T cells) are then used to elicit beta cell destruction and diabetes.

OT-I C57BL/6 mice carrying CD8+ T cells towards a class I-restricted epitope of ovalbumin were treated by administration of epitope (SIINFEKL, SEQ ID NO:2). In the tested group, such administration was carried out by the subcutaneous (SC) route with a formulation including 100 µg of peptide in aluminium hydroxide and a reducing compound made of glutathione (50 µM) and NADPH (50 µM). In a control group, the same procedure was used but without glutathione and NADPH.

After 3 injections made at an interval of 10 days, mice were sacrificed and individual splenocyte populations were prepared. CD8+ T cells were prepared after 2 cycles of stimulation *in vitro* and characterized. Cells obtained from the control group show signs of activation and cytotoxic potential, including expression of CD103 and positive intracellular staining for granzyme B and perforin. In contrast, cells obtained from mice immunized in the presence of glutathione and NADPH express markers such as CTLA2 and TIGIT, indicating their suppressive phenotype.

Individual preparations of CD8+ T cells were administered (50x10³ cells) by the intravenous (IV) route in RIP-OVA mice, which express OVA in the pancreatic islets. All mice reconstituted with CD8+ T cells from the control group rapidly developed diabetes as assessed by glycemia. A minority of mice receiving CD8+ T cells from OT-1 mice treated with the formulation containing glutathione and NADPH develop a delayed and mild form of diabetes.

It is therefore concluded that activating CD8+ T cells in the presence of a mix of glutathione and NADPH is sufficient to drastically reduce the cytotoxic potential of such cells in the context of insulin-dependent diabetes.

### Example 3

### Prevention of myasthenia gravis in a mouse model

Myasthenia gravis (MG) is characterized by an autoimmune attack of the neuromuscular junction leading to progressive muscle weakness and difficulty to breathe. Pathogenic antibodies produced in the framework of an autoimmune reaction are directed towards various components of the neuromuscular junction, including the nicotinic acetylcholine receptor (nAchR), LRP4, Musk and agrin.

Experimentally, MG can be induced in rats or mice by immunization with Torpedo fish acetyl-choline receptor in so far as antibody produced against this receptor cross-react with the rat or mouse receptor.

The sequence of the mouse nAchR contains an epitope which is presented by the MHC-like CDld molecule. Such epitope has the sequence FAI VKF TKV LL (100-110: SEQ ID NO:3).

A group of control C57BL/6 mice is treated by 2 injections of 100 µg of peptide of SEQ ID NO:3 adsorbed on aluminium hydroxide by the intraperitoneal (IP) route (a body compartment rich in NKT cells), at an interval of 10 days. A second group is treated by the same protocol but with addition of GILT (gamma interferon-inducible lysosomal thiol reductase, 50 µM) in the formulation.

Ten days after the last IP injection, mice of both groups are immunized by the subcutaneous route with 20 µg of Torpedo AchR emulsified in Freund's adjuvant. One additional injection of 20 µg is made after 4 weeks, using incomplete Freund's adjuvant.

Six weeks after the last injection of the Torpedo AchR the first signs of muscle weakness are observed and graded according to a score from 0 (normal); 1 (weakness after exercise, reduced mobility); 2 (weakness at rest) or 3 (moribund, dehydrated and paralyzed) . It is shown that the mice treated with the control formulation develop scores spread in between 2 and 3, whilst mice treated with the formulation containing GILT show score of 0 or 1.

Serum was collected from individual mice at the end of the observation period (3 months, except for mice scoring 3 which are sacrificed as soon as they reached that score) for evaluation of specific antibodies to the AchR. This is carried out in an ELISA using Torpedo AchR to coat polystyrene plates and incubation with serial dilutions of individual serums. A mean concentration of 200 µg of total IgG antibodies per ml serum is observed in the control group, as compared to 12 µg of total IgG in the group of mice pre-immunized with peptide of SEQ ID NO:3 in the presence of GILT.

### Example 4

### Prevention of anti-gliadine immune response in the context of experimental celiac disease

Celiac disease results from an autoimmune response towards epitopes from gliadine, a component of gluten. In particular, epitope 57-73 of alpha-gliadine fragment alpha-1/alpha-2 (QLQ PFP QPE LPY PQP QS, SEQ ID NO:4) is deamidated in position 4 (core sequence underlined) by transglutaminase in the presence of calcium, which confers a higher affinity for human DQ2.5 HLA molecule. This leads to activation of class II-restricted T cells and inflammation in intestinal mucosa at the origin of celiac disease symptoms.

There is no straightforward mouse model for such disease. However, several transgenic models have been described which are suitable to explore at least parts of immune pathology and define potential novel therapies. Transgenic mice expressing the human DR3-DQ2.5 MHC haplotype can be utilized to demonstrate whether tolerance to gliadine epitopes can be obtained.

In order to mimic human situation wherein peptide of SEQ ID NO:4 is naturally deamidated by the enzyme tissue transglutaminase, a deamidated version of the peptide is used wherein glutamine (Q) is replaced by a charged glutamate residue (E) at position 7.

SEQ ID NO:5 QLQ PFP EPE LPY PQP QS
C57BL/6 DR3-DQ2 transgenic mice are immunized using 50 µg of peptide of SEQ ID NO:5 emulsified in Freund's adjuvant and injected in the footpath. A second injection of 50 µg in incomplete Freund's adjuvant is made 2 weeks later. A month later, mice are killed and the splenocytes prepared for a T cell stimulation assay. To this end, CD4+ T cells from the splenocyte population are prepared by FACS sorting using specific anti-CD4+ antibodies. These are then incubated in the presence of dendritic cells loaded with the peptide used for immunization (SEQ ID NO:5), and the presence of peptide specific CD4+ T cells is detected after 1 week in culture. A second group of mice is treated the same way, but at the end of the immunization period, the peptide is injected in the ear skin and the development of a local swelling reaction after 3 days is read as the presence of a delayed type hypersensitivity reaction.

The experimental group of mice is first treated by subcutaneous injections of peptide of SEQ ID NO:5, using 100 µg mixed with aluminium hydroxide and 100 µM of glutathione. Four of such injections are made at intervals of 10 days. Fifteen days after the last injection, a footpath immunization procedure as for the control group is initiated. It is shown that CD4+ cells from splenocytes do not proliferate in the presence of the deamidated version of peptide of SEQ ID 5. Moreover, testing for delayed type reaction remains negative in this group.

It is therefore concluded that immunization with a gliadine epitope in the presence of a reducing agent is sufficient as to induce tolerance to such epitope even in the context of an active systemic (footpath) immunization with strong adjuvant.

### Example 5

### Prevention of immunization towards coagulation factor VIII administered by the subcutaneous route

The development of antibodies to coagulation factor VIII still constitutes a major side effect in the treatment of haemophilia A patients. Such antibodies have the potential to neutralize the functional activity of factor VIII (called inhibitor antibodies), thereby putting patients at risk of severe bleeding.

Factor VIII is immunogenic, as characterized by both an innate and an adaptive immune responses. Patent WO2012/069575 describes methods by which deleting factor VIII epitopes presented by the MHC-like CDld molecule eliminates the risk of inducing inhibitor antibodies.

However, recent developments in the therapy of haemophilia A patients deal with factor VIII formulation for subcutaneous administration instead of intravenous. The subcutaneous route is more immunogenic than the IV route, due to the presence of a high density of antigen-presenting cells, including macrophages and dendritic cells.

A pegylated form of human recombinant (r) factor VIII was used for subcutaneous administration in factor VIII KO mice, at a dose of 100 IU/kg twice a week for a total of 6 weeks.

A control group of haemophilia A mice received the preparation of factor VIII (GenBank accession reference: AAA52484.1; SEQ ID NO:6), whilst the tested group received the same preparation in which 200 µM of glutathione has been added. After 6 weeks, mice are bled to determine the concentration of anti-factor VIII antibodies by solid-phase ELISA and that of inhibitors using a commercially available chromogenic assay. Results for antibodies are expressed in arbitrary units/ml established by reference to the level of fluorescence obtained by serial dilutions of a factor VIII-specific monoclonal antibody. Results for the inhibitor assay are expressed in Bethesda units/ml.

It is shown that mice injected with rFactor VIII have produced a mean of 750 µg/ml of anti-factor VIII antibodies and a titer of inhibitors of 1200 BU/ml. Mice under treatment with the glutathione-containing factor VIII preparation show a mean of 150 µg/ml of anti-factor VIII antibodies and a titer of 225 BU/ml for inhibitors.

It is therefore concluded that addition of a reducing compound to the factor VIII formulation is sufficient as to significantly reduce the factor VIII specific immune response. As the immune response towards Factor VIII includes sequential activation of specific NKT cells, it is additionally concluded that the reducing compound has the capacity to prevent specific NKT cell activation.

### Example 6

### Long term evaluation of the toxicity of antibodies to TNF-alpha in a mouse model

Antibodies to tumor necrosis factor (TNF) alpha are commonly used for the treatment of a number of chronic inflammatory diseases (heavy chain Fab fragment, SEQ ID NO:7; light chain Fab fragment SEQ ID NO:8), such as rheumatoid arthritis. Although efficient in a majority of patients, the recurrent administration of these antibodies is poised by an increased risk of infection and development of tumors. Today, there is no possibility to identify patients who are at risk of developing such complications, due, inter alia to the fact that commercially available anti-TNF-alpha antibodies do not suppress all activities of TNF-alpha, yet do suppress the binding of TNF to TNF receptor 2 (TNFR2), which is required for regulatory T cell activation. Besides, the concentration of TNF-alpha shows considerable variations in between patients, due to persistence of TNF-alpha/anti-TNF-alpha complexes in the presence or absence of anti-antibodies.

An animal model by which it would be possible to predict the long term outcome of anti-TNF alpha antibody administration would be of much help, including its amenability to genetic manipulation to evaluate TNF alpha on single targets. However, administration of anti-TNF alpha antibodies in animal is rapidly followed by an immune response preventing any long term assessment of effects. This is particularly relevant for the subcutaneous administration mimicking clinical use.

Mice of the C57BL/6 strain are treated by injection of 50 µg of anti-TNF-alpha antibody, 4 injections made at one week interval. A control group receives the formulation of antibody as used in the clinic, whilst a second group of mice received the same formulation but with addition of 50 µM glutathione for each injection.

Four weeks after the last injection, it is shown that the level of circulating complexes of TNF-alpha and anti-TNF-alpha antibody remains at high levels (mean of 200 ng/ml) in the group of mice treated by the glutathione-containing formulation, whilst drastically reduced concentrations (mean of 7 ng/ml) of TNF-alpha/anti-TNF-alpha antibodies are measured in the control group.

This results is interpreted as depicting a rapid clearance of anti-TNF-alpha/TNF-alpha complexes from the circulation by the induction of anti-antibodies in the control group. This conclusion is confirmed by the detection of anti-antibodies in a solid-phase ELISA in which anti-TNF-alpha antibodies are used to coat the plates, followed by a dilution of mouse serum and detection of mouse antibodies bound to human anti-TNF-alpha antibodies. A mean of 6 arbitrary units/ml is seen in the group receiving the glutathione formulation and a mean of 145 units/ml is calculated in the control group.

Therefore, addition of a reducing compound to the anti-TNF-alpha antibody formulation is effective in drastically reducing its immunogenicity.

## Claims

1. A pharmaceutically compatible antioxidant for use in the treatment or the prevention of an unwanted immune response.

2. The pharmaceutically compatible antioxidant of claim 1, being present in a pharmaceutical composition or incorporated in a pharmaceutical kit of part, further comprising a pharmaceutical peptide molecule, wherein the said pharmaceutical peptide molecule is preferably selected from the group of antigens associated to autoimmune and/or chronic inflammatory diseases, injectable biologicals and epitopes being part of the said biologicals.

3. The pharmaceutically compatible antioxidant of claim 1 or of claim 2 for use in the treatment of autoimmune diseases, preferably together with a vaccine adjuvant, or in inducing tolerance to peptide-based biologicals.

4. The pharmaceutically compatible antioxidant of claim 1, or the pharmaceutical composition or kit of parts of claim 2 or claim 3 being for administration by the subcutaneous route.

5. A vaccine composition comprising a peptide-based antigen and a pharmaceutically compatible antioxidant.

6. The vaccine composition of claim 5 being for use in the treatment of autoimmune diseases, preferably selected from the group consisting of type 1 diabetes, chronic inflammatory demyelinating neuropathies (such as multiple sclerosis), diseases of the neuro-muscular junction (such as myasthenia gravis), diseases of the thyroid (such as Hashimoto's and Grave's diseases), inflammatory diseases of the bowel including Crohn's disease, ulcerative rectocolitis and celiac disease.

7. The vaccine composition of claims 5 or 6 being for local injection, and wherein the pharmaceutically compatible antioxidant is in an amount sufficient for imparting reducing conditions in the extracellular medium of the injection site.

8. The vaccine composition according to any one of the preceding claims 5 to 7 further comprising a vaccine adjuvant, preferably selected from the group consisting of bacterial lipopolisaccharides, CpG oligonucleotides, double-stranded RNA and aluminium hydroxide.

9. An *ex vivo* method for eliciting suppressive antigen-specific T lymphocytes being CD4, CD8 and/or NKT comprising the step of putting *ex vivo* T lymphocyte in contact to a specific antigen, under reducing conditions and preferably selecting the treated lymphocytes with higher surface expression of a molecule selected from the group consisting of TIGIT, DLL4 and CTLA2, and/or with higher secretion of a molecule selected from the group consisting of IL-13, IL-10, prostaglandin E2, TGF-beta, amphiregulin, MMP9 and ADAM33.

10. T lymphocytes obtainable by the method of claim 9.

11. A pharmaceutical composition comprising the T lymphocytes of claim 10.

12. The antioxidant according to any one of the preceding claims being present at a concentration comprised between 0.1 µM and 5 mM, preferably between 0.3 µm and 1 mM, more preferably between 1 µM and 0.3 mM, still more preferably between 3 µM and 100 µM, or between 5 µM and 50 µM.

13. The antioxidant according to any one of the preceding claims being selected from the group consisting of N-acetyl cysteine, glutathione, thioredoxin, thioredoxin derivatives, glutaredoxin, peroxiredoxin and gamma interferon-inducible lysosomal thiol reductase (GILT), and mixtures thereof.

14. The antioxidant of claim 13, further comprising NADH and/or NADPH, advantageously at a concentration comprised between 0.1 µM and 5 mM, preferably between 0.3 µm and 1 mM, more preferably between 1 µM and 0.3 mM, still more preferably between 3 µM and 100 µM, or between 5 µM and 50 µM.

15. The antioxidant of claims 13 or 14 further comprising thioreductase.
